(19) Europäisches Patentamt
European Patent Office
Office européen des brevets

(11) Publication number: **0 379 351 A1**

# (12) EUROPEAN PATENT APPLICATION

(21) Application number: **90300478.6**

(51) Int. Cl.5: **C07D 401/12, A61K 31/435**

(22) Date of filing: **17.01.90**

(30) Priority: **17.01.89 CA 588458**

(43) Date of publication of application:
**25.07.90 Bulletin 90/30**

(84) Designated Contracting States:
**AT BE CH DE DK ES FR GB GR IT LI LU NL SE**

(71) Applicant: **SYNPHAR LABORATORIES INC.**
**24, 4290 - 91 A Street**
**Edmonton Alberta T6E 5V2(CA)**

(72) Inventor: **Daneshtalab, Mohsen**
**10419 - 10 A Avenue**
**Edmonton, Alberta T6J 6G2(CA)**
Inventor: **Micetich, Ronald G.**
**12 Braeside Terrace, Sherwood Park**
**Alberta T8A 3V6(CA)**
Inventor: **Nguyen, Dai Quoc**
**12344 - 50 Street, Edmonton**
**Alberta T5W 3E1(CA)**

(74) Representative: **Pidgeon, Robert John et al**
**Appleyard Lees & Co. 15 Clare Road**
**Halifax West Yorkshire HX1 2HY(GB)**

## (54) 1,4-Dihydropyridine derivatives.

(57) Novel therapeutic 1,4-dihydropyridine derivatives of the formula

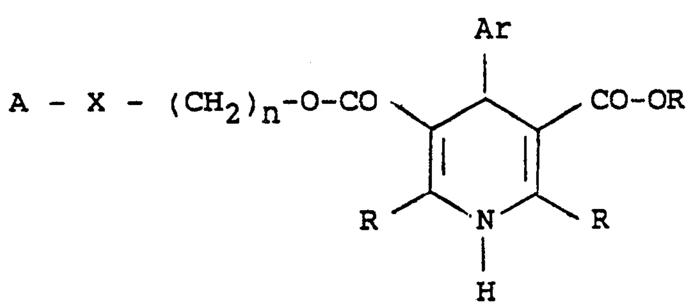

(I)

in which:
A is an optionally substituted non-fused azole moiety;
R is a lower alkyl group;
X is $-CH_2-$ , -S- , -SO- or $-SO_2-$ ;
n is 5, 6, 7 or 8; and
Ar is a phenyl group substituted once or twice by $NO_2$, $CF_3$ or Cl groups.

EP 0 379 351 A1

# 1,4-DIHYDROPYRIDINE DERIVATIVES

The present invention relates to novel 1,4-dihydropyridine compounds, to processes for their preparation, to their use as therapeutic agents, in particular as cardiovascular regulating agents, to the formulation in pharmaceutically acceptable compositions, and to certain intermediate compounds therefor, and their preparation.

It is well known that numerous 1,4-dihydropyridine derivatives exhibit calcium channel blocking (or antagonist) antihypertensive activity.

Exemplary compounds of this type have been described in Japanese Patent 60,226,876 [85,226,876] (11, 1985); Japanese Patent 5978,185 [8478, 185] (5, 1984); Japanese Patent 6097,955 [8597, 955] (5, 1985); French Patent 2,511,370 (3, 1983) and EP 151,006 [8, 1985).

Of particular interest is the disclosure of D. J. Tiggle et al. entitled "Dimeric 1, 4-Dihydropyridines as Calcium Channel Antagonists" in J. Med. Chem. 1988, 31, 1489 - 1492.

The invention comprises a 1,4-dihydropyridine derivative of the formula

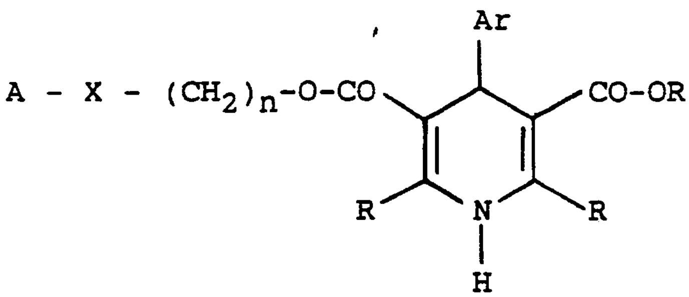

(I)

in which:

A is an optionally substituted non-fused azole moiety;

R is a lower alkyl group;

X is $-CH_2-$, -S-, -SO- or $-SO_2-$;

n is 5, 6, 7 or 8; and

Ar is a phenyl group substituted once or twice by $NO_2$, $CF_3$ or Cl groups.

Optional substituent(s) of a non-fused azole moiety may suitably be lower alkyl group(s), especially methyl. Preferably, A is a non-fused azole moiety, for example an isoxazolyl, pyrazolyl or thiazolyl group, substituted by 1 or 2 methyl groups.

The phenyl group Ar may be substituted by two different groups selected from $NO_2$, $CF_3$ or Cl, or, more usually, by the same groups. Most preferably the group Ar has only one substituent, $NO_2$ being particularly preferred.

Preferred sites for substituent(s) of the group Ar are the 2- and/or the 3- positions.

The symbol n may particularly suitably represent 5 or, most preferably, 6.

Preferably, each group R is the same, and is a methyl group.

One preferred group X is $-CH_2-$.

Another preferred group X is $-SO_2-$.

Preferably, the azole moiety has 5 ring atoms, most suitably a nitrogen atom, another hetero atom selected from oxygen, sulphur and nitrogen, with the remainder being carbon atoms. Preferably, there is a nitrogen atom at the 2- position relative to the ring linkage. Suitably, there may be a substituent, preferably a methyl group, at the 3- position relative to the linkage.

Preferably, the azole moiety is aromatic.

In a first preferred embodiment there is provided a 1,4,dihydropyridine derivative of the formula II

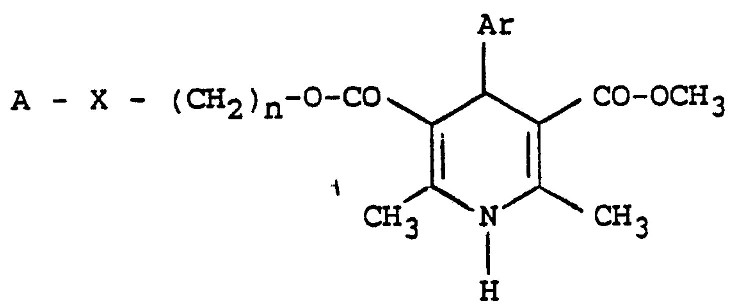

(II)

where
A is 3-methyl-5-isoxazolyl; 3,5-dimethyl-1-pyrazolyl, or 4-methyl-2-thiazolyl;
X is a $-CH_2-$, -S-, -SO- or $-SO_2-$;
n is 5, 6, 7 or 8; and
Ar is a phenyl group substituted once or twice by $NO_2$, $CF_3$, or Cl groups.

In a second preferred embodiment, the pharmaceutical compound has the structural formula II in which A is 4-methyl-2-thiazolyl; X is methylene; n is 6; and Ar is a phenyl group substituted once by $NO_2$.

In a third preferred embodiment the pharmaceutical compound has a structural formula II in which A is 3-methyl-5-isoxazolyl; X is methylene; n is 6; and Ar is a phenyl group substituted once by $NO_2$.

In a fourth preferred embodiment, the pharmaceutical compound has the structural formula (II) wherein A is 4-methyl-2-thiazolyl; X is sulfone; n is 6; and Ar is a phenyl group substituted once by $NO_2$.

It has been found that physiologically acceptable compounds of the formula I and II possess valuable pharmacological properties. More particularly, they exhibit specific calcium channel blocking activity. Advantageously, such compounds exhibit no effect, or in some instances, positive ionotropic effect on the heart muscle without altering the heart rate and produce a relaxation effect on smooth muscle. Furthermore, it has been discovered that such compounds exhibit angiotensin inhibitory activity in addition to their calcium channel blocking activity. That such compounds possess both of these properties together is not only most beneficial but also somewhat surprising because the controlling mechanisms for angiotensin release and high blood pressure control are different.

Thus, the compounds of the formulae I and II can be used as active compounds in medicaments.

The compounds of formulae I and II can have an asymmetric carbon at the C4 position of the 1,4-dihydropyridine ring. Thus they can exist as racemates in various optically active forms.

In another aspect of the invention, there is provided a process for the preparation of the compounds of the formulae I and II which process comprises reacting a compound of formula III

$A-X-(CH_2)_n-O-CO-CH_2-O-R$ (III)

wherein A, X and n are as defined by any of the preceding claims, with an alkyl-$\beta$-aminoalkylcrotonate and a compound of formula IV

Ar-CHO (IV)

and optionally oxidising a resultant compound of formula I or II in which -X- is -S- or -SO- to generate a higher oxide.

For example, to make compounds of formula I in which each R is methyl, Ar is mono- or di-substituted, A is one of the preferred azolyl groups mentioned above, and X represents $-CH_2-$, a heterocyclic alkyl acetoacetate, of the general formula III

$A-CH_2-(CH_2)_n-O-CO-CH_2-CO-CH_3$ (III)

in which A is as defined, and is for example 3-methyl-5-isoxazolyl, 3,5-dimethyl-1-pyrazolyl, or 4-methyl-2-thiazolyl and n has a value of between 5 to 8, is reacted with methyl-$\beta$-aminocrotonate and a substituted benzaldehyde IV, suitably a benzaldehyde of the general formula IV

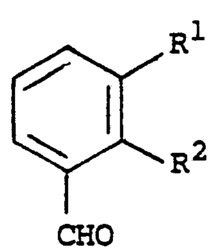

(IV)

in which, preferably, $R_1$ is a hydrogen, nitro, trifluoromethyl or chloro, $R_2$ is a hydrogen, nitro, trifluoromethyl or chloro, or $R_1 = R_2$ and is a chloro group. The reaction is carried out in a suitable solvent.

As a further example of the general process, the compounds having the general formula I and II, wherein X comprises sulfur, sulfoxide or sulfone, may be prepared by reacting a mercapto, sulfoxy, or sulfonyl alkyl acetoacetate having the general formula III

$A\text{-}X\text{-}(CH_2)_n\text{-}OCOCH_2COCH_3$ (III)

in which A is as defined, and is most suitably 4-methyl-2-thiazolyl, X is sulfur, sulfoxy or sulfone, and n has a value of 5 to 8, with methyl-$\beta$-aminocrotonate and a benzaldehyde having the general formula IV in a suitable solvent.

A suitable solvent is an inert organic solvent, for example an alcohol, such as isopropanol.

The compounds of the general formula III may be prepared by reacting diketene, or a lower alkyl-substituted diketene, in an inert solvent, with an $\omega$-alkanol having the general formula V

$A\text{-}X\text{-}(CH_2)_q\text{-}CH_2OH$ (V)

in which A and X are as defined in any of the statements above, and q is from 4 to 7.

The compounds having the general formula V in which -X- represents $-CH_2-$ are suitably prepared by reacting the appropriate lithium salt of, for example, 3,5-dimethyl isoxazole, 3,5-dimethylpyrazole or 2,4-dimethyl thiazole with, for example, 6-bromo-1-hexanol in an inert solvent, preferably at a temperature in the range of about -70° C.

The compounds having the general formula V in which -X- represents -S- are suitably prepared by reacting an appropriate sodium salt of a mercapto derivative of an appropriate heterocycle comprising, for example, 4-methyl-2-thiazolyl with, for example, 6-bromo-1-hexanol in an inert solvent. The resultant thioether derivative of general formula III may, if desired, be consecutively oxidized utilizing, for example, m-chlorobenzoic acid to the related sulfoxy and by potassium permanganate in acetic acid to the sulfonyl analogue. Likewise, compounds of formula I wherein -X-is -S- may be oxidised.

Another broad aspect of the invention is a process for the preparation of the compounds of formula I or II comprising: reacting the compound of formula III in which A is 3-methyl-5-isoxazolyl, 3,5-dimethyl-1-pyrazolyl, or 4-methyl-2-thiazolyl, and n has a value of between 5 to 8 with methyl-$\beta$-aminocrotonate and a mono-or disubstituted benzaldehyde of the formula IV in which $R_1$ is a hydrogen, nitro, trifluoromethyl, or chloro, or $R_1 = R_2$ and is a chloro.

The compounds of this invention of formulae I and II are suitably prepared, applying modified Hantzsch pyridine synthesis, by reacting the appropriately substituted heterocyclic alkyl acetoacetate or substituted heterocyclic mercapto alkyl acetoacetate with a substituted benzaldehyde derivative and methyl-$\beta$-aminocrotonate in isopropanol. The compounds of formula III are suitably prepared by reacting diketene or an alkyldiketene with an appropriately substituted heterocyclic $\omega$-alkanol or substituted heterocyclic mercapto $\omega$-alkanol in benzene. The related sulfoxy and sulfonyl derivatives of formula VI may be prepared by oxidizing the relative mercapto derivative with m-chloroperbenzoic acid in $CH_2Cl_2$ followed, if required, by further oxidation with potassium permanganate in acetic acid. All the products in this invention are characterized by their respective nmr, ir spectra and their elemental analysis. The relative purity has been determined by HPLC. Calcium antagonistic activity of the compounds was determined at the concentration required to produce 50% inhibition of the muscarinic receptor-mediated $Ca^{2+}$-dependent contraction of guinea pig ileal longitudinal smooth muscle assay.

The compounds of formula II, wherein $R_1$ and $R_2$ were chloro, trifluoromethyl, nitro; $R_3$ is a substituted aromatic five-membered heterocycle selected from the group consisting of 3-methyl-5-isoxazolyl; 3,5-dimethyl-1-pyrazolyl and 4-methyl-2-thiazolyl, n has a value of 5 to 8, and X is $CH_2$ were prepared as follows.

An equimolecular mixture of an appropriately substituted aromatic five-membered heterocyclic alkyl acetoacetate of the formula III, a mono or disubstituted benzaldehyde of formula IV, and methyl-$\beta$-aminocrotonate in isopropanol were refluxed for a period of 12 - 36 hrs. The crude products were purified by elution from a silica gel column using an appropriate eluant.

The alkyl acetoacetates could, for example, be 5-(7-acetoacetoxyhept-1-yl)-3-methylisoxazole; 1-(6-acetoacetoxyhex-1-yl)-3,5-dimethylpyrazole; 2-(7- acetoacetoxyhept-1-yl)-4-methylthiazole; 2-(6-acetoacetoxyhexylthio)-4-methylthiazole; or 2-(6-acetoacetoxyhexylsulfonyl)-4-methylthiazole. The benzaldehyde could, for example, be selected from those described supra.

The acetoacetate derivatives of formula III in which -X- is $-CH_2-$ were prepared by refluxing an equivalent mixture of diketene and appropriately substituted aromatic five-membered heterocyclic -alkanol of the formula V, in benzene for a period of 8 -12 h. The products were purified by vacuum distillation in yields varying from 50 - 80%.

The heterocyclic $\omega$-alkanols could, for example, be 7-(3-methylisoxazol-5-yl)heptan-1-ol; 6-(3,5-dimethylpyrazol-1-yl)hexan-1-ol; 7-(4-methylthiazol-2-yl)heptan-1-ol or 6-(4-methylthiazol-2-yl)thiohexan-1-ol.

The ω-hydroxyalkyl heterocyclic systems of the formula V were prepared through the lithiation of the appropriately substituted aromatic five-membered heterocycle followed by reaction with 6-bromo-hexanol, in THF at -70 °C. The crude products were purified by elution from a silica gel column using different ratio of $CH_2Cl_2$-hexane. The yield varied from 50 - 70%.

The compounds of formula II wherein $R_1$ and $R_2$ are chloro, trifluoromethyl, nitro; $R_3$ is a substituted aromatic five-membered heterocycle selected from the group consisting of 3-methyl-5-isoxazolyl; 3,5-dimethyl-1-pyrazolyl and 4-methyl-2-thiazolyl, n has a value of 5 to 8, and X is sulfur, sulfoxy or sulfone, and n has a value of 4 to 7 have been prepared by the following method.

An equimolecular mixture of an appropriately substituted aromatic heterocyclic mercapto, sulfoxy, or sulfonyl alkyl acetoacetate of formula III, mono or disubstituted benzaldehyde of formula IV, and methyl-β-aminocrotonate in isopropanol was refluxed for a period of 12 - 36 hrs. The crude products were purified by silica gel column chromatography in yields varying from 70 - 80%.

The acetoacetates of formula III could, for example, be 5-(7-acetoacetoxyhept-1-yl)-3-methylisoxazole; 1-(6-acetoacetoxyhex-1-yl)-3,5-dimethylpyrazole; 2-(7-acetoacetoxyhept-1-yl)-4-methylthiazole; 2-(6-acetoacetoxyhexylthio)-4-methylthiazole; or 2-(6-acetoacetoxyhexylsulfonyl)-4-methylthiazole. The benzaldehydes could be selected from those described supra.

The substituted aromatic heterocyclic mercapto, sulfoxy or sulfonyl alkyl acetoacetates of formula III were prepared by refluxing an equimolecular mixture of substituted aromatic heterocyclic mercapto alkanol of formula V with diketene in benzene for a period of 12 h. The crude product was purified by elution from a silica gel column using $CHCl_3$ as an eluant, in almost quantitative yield. The resulting product was oxidized by m-chloroperbenzoic acid (MCPBA) in $CH_2Cl_2$ at room temperature overnight, followed by purification through elution in a silica gel column using $CHCl_3$ as an eluant, to get the related sulfoxy derivative in 68% yield. The sulfoxy derivative was further oxidized to the sulfonyl derivative using potassium permanganate in acetic acid for 4 h. The product was purified by silica gel column chromatography using chloroform-hexane (80:20) in 60% yield.

The ω-alkanol of structure V could, for example, be 7-(3-methylisoxazol-5-yl)heptan-1-ol; 6-(3,5-dimethylpyrazol-1-yl)hexan-1-ol; 7-(4-methylthiazol-2-yl)heptan-1-ol or 6-(4-methylthiazol-2-yl)thiohexan-1-ol.

The substituted aromatic heterocyclic mercapto ω-alkanol of structure V was prepared by refluxing an equimolecular mixture of an appropriately substituted mercapto heterocycle with 6-bromo-hexanol and potassium carbonate in acetone for 14 h. The crude product was purified by elution from a silica gel column using ethylacetate-methanol (99:1) in quantitative yield.

The selected compounds of this invention were tested for their calcium channel blocking activity and other possible pharmacological activity under a wide-range screening program.

More particularly, 3-[7-(3-methylisoxazol-5-yl)heptyloxycarbonyl]-5-methoxycarbonyl-2,6-dimethyl-4-(3-nitrophenyl)-1,4-dihydropyridine; 3-[7-(3-methylisoxazol-5-yl)heptyloxycarbonyl]-5-methoxycarbonyl-2,6-dimethyl-4-(3-trifluoromethylphenyl)-1,4-dihydropyridine; 3-[6-(3,5-dimethylpyrazol-1-yl)hexyloxycarbonyl]-5-methoxycarbonyl-2,6-dimethyl-4-(3-nitrophenyl)-1,4-dihydropyridine; 3-[7-(4-methylthiazol-2-yl)-heptyloxycarbonyl]-5-methoxycarbonyl-2,6-dimethyl-4-(3 nitrophenyl)-1,4-dihydropyridine; the 3-[7-(4-methylthiazol-2-yl)heptyloxycarbonyl]-5-methoxycarbonyl-2,6-dimethyl-4-(3-trifluoromethylphenyl)-1,4-dihydropyridine; the 3-[7-(4-methylthiazol-2-yl)heptyloxycarbonyl]-5-methoxycarbonyl-2,6-dimethyl-4-(3-chlorophenyl)-1,4-dihydropyridine; the 3-[7-(4-methylthiazol-2-yl)heptyloxycarbonyl]-5-methoxycarbonyl-2,6-dimethyl-4-(2,3-dichlorophenyl)-1,4-dihydropyridine; 3-[6-(4-methylthiazol-2-yl)-thiohexyloxycarbonyl]-5-methoxycarbonyl-2,6-dimethyl-4-(3-nitrophenyl)-1,4-dihydropyridine; the 3-[6-(4-methylthiazol-2-yl)-sulfoxyhexyloxycarbonyl]-5-methoxycarbonyl-2,6-dimethyl-4-(3-nitrophenyl)-1,4-dihydropyridine; 3-[6-(4-methylthiazol-2-yl)-sulfonylhexyloxycarbonyl]-5-methoxycarbonyl-2,6-dimethyl-4-(3-nitrophenyl)-1,4-dihydropyridine were tested for their calcium channel blocking activity.

Almost all the compounds tested were shown to possess very strong calcium channel blocking activity on smooth muscles and to lack negative ionotropic effect on the heart. At the concentrations which normally exhibit their Ca- blocking effect in smooth muscles, some of them have been shown to increase the heart contractile force, for example, 3-[7-(4-methylthiazol-2-yl)heptyloxycarbonyl]-5-methoxycarbonyl-2,6-dimethyl-4-(3-nitrophenyl)-1,4-dihydropyridine and 3-[6-(4-methylthiazol-2-yl) sulfonylhexyloxycarbonyl]-5-methoxycarbonyl-2,6-dimethyl-4-(3-nitrophenyl)-1,4-dihydropyridine, without changing the heart rate.

More particularly, the structure 3-[7-(4-methylthiazol-2-yl)heptyloxycarbonyl]-5-methoxycarbonyl-2,6-dimethyl-4-(3-nitrophenyl)-1,4-dihydropyridine, which has a relative potency of 4.3 ($IC_{50}$ 3.25 +/- 0.33 x $10^{-9}$M) to compare with Nifedipine * ($IC_{50}$ 1.40 +/- 0.20 x $10^{-8}$M) as a calcium antagonist in smooth

*** Trade Mark**

muscle, shows about 12% positive ionotropic effect on the heart. In the same manner, compound 3-[6-(4-methylthiazol-2-yl)sulfonylhexyloxycarbonyl]-5-methoxycarbonyl-2,6-dimethyl-4-(3-nitrophenyl)-1,4-dihydropyridine which has a relative potency of 2.5 as compared with Nifedipine ($IC_{50}$ 5.57 +/- 0.00 x $10^{-9}$M) on smooth muscle, shows about 22% positive ionotropic effect on heart muscle, in the same concentration. Mode of action of 3-[7-(4-methylthiazol-2-yl)heptyloxycarbonyl]-5-methoxycarbonyl-2,6-dimethyl-4-(3-nitrophenyl)-1,4-dihydropyridine is similar to 3-ethoxycarbonyl-5-methoxycarbonyl-4-(2,3-dichlorophenyl)-2,6-dimethyl-1,4-dihydropyridine (Felodipine, H 154/82; J. Cardiovascular Pharmacology, 1987, 10 (Suppl. 1) S60 - S65), a compound in third phase of clinical trials by Astra, from potency, with regard to its antihypertensive property. But it is better than Felodipine with regard to its cardiac effect and duration of action. An increase in the cardiac contractile force in 3-[7-(4-methylthiazol-2-yl)heptyloxycarbonyl]-5-methoxycarbonyl-2,6-dimethyl-4-(3-nitrophenyl)-1,4-dihydropyridine brings an advantage over Felodipine which shows no effect on the heart in the concentration of its smooth muscle calcium channel blocking effectiveness.

Upon general pharmacological screening, the selected compounds of this invention show other activities. More particularly, structure 3-[7-(3-methylisoxazol-5-yl)heptyloxycarbonyl]-5-methoxycarbonyl-2,6-dimethyl-4-(3-nitrophenyl)-1,4-dihydropyridine and structure 3-[7-(4-methylthiazol-2-yl)heptyloxycarbonyl]-5-methoxycarbonyl-2,6-dimethyl-4-(3-chlorophenyl)-1,4-dihydropyridine have shown very strong angiotensin inhibitory, $LTD_4$ antagonistic, anti-ulcer stressic activities; 3-[6-(3,5-dimethylpyrazol-1-yl)hexyloxycarbonyl]-5-methoxycarbonyl-2,6-dimethyl-4-(3-nitrophenyl)-1,4-dihydropyridine and structure 3-[7-(4-methylthiazol-2-yl)heptyloxycarbonyl]-5-methoxycarbonyl-2,6-dimethyl-4-(3-trifluoromethylphenyl)-1,4-dihydropyridine possessed very strong anti-inflammatory activity in addition to the other activities mentioned for the structure 3-[7-(3-methylisoxazol-5-yl)heptyloxycarbonyl]-5-methoxycarbonyl-2,6-dimethyl-4-(3-nitrophenyl)-1,4-dihydropyridine.

The invention will now be further described with reference to the following Examples of the preparation of compounds of formula I and intermediates therefor.

## Example 1

3-[7-(3-methylisoxazol-5-yl)heptyloxycarbonyl]-5-methoxycarbonyl-2,6-dimethyl-4-(3-nitrophenyl)-1,4-dihydropyridine. (1)

$R_1$ = 3-methylisoxazol-5-yl; n = 6; $R_2$ = m-$NO_2$; X = $CH_2$.

(See schematic representation of reaction given herebelow)

7-(3-methylisoxazol-5-yl)heptylacetoacetate (1.967g, 0.007 mol) was added to a solution of methyl-$\beta$-aminocrotonate (0.805g, 0.007 mol) and m-nitrobenzaldehyde (1.057g, 0.007 mol) in isopropanol (35mL). The reaction mixture was heated under reflux for 12h and then concentrated under reduced pressure. The residual oil was purified by elution from a silica gel column using dichloromethane-hexane (9:1, v/v) as eluant to give 3-[7-(3-methylisoxazol-5-yl)heptyloxycarbonyl]-5-methoxycarbonyl-2,6-dimethyl-4-(3-nitrophenyl)-1,4-dihydropyridine (60%) as a pale yellow thick oil. IR (neat) 3330 (NH) and 1701 and 1676 ($CO_2R$)$cm^{-1}$, NMR ($CDCl_3$) 1.20 - 1.40 (m, 6H, -$(CH_2)_3$), 1.56 - 1.74 (m, 4H, -$(CH_2)_2$), 2.30 (S, 3H, Me), 2.39 (S, 3H, Me), 2.72 (t, J = 9Hz, 2H, $CH_2$-Het), 3.70 (S, 3H, $CO_2Me$), 3.99 - 4.16 (m, 2H, -$OCH_2$), 5.14 (S, 1H, H-4), 5.89 (S, 1H, Het-H), 7.06 (S, 1H, NH), 7.38 - 8.14 (m, 4H, H-2′, H-4′, H-5′, H-6′). Analysis found: C, 63.48; H, 6.45,. N, 7.99. Required: C, 63.39; H, 6.50; N, 8.21 ($C_{27}H_{33}N_3O_7$).

Schematic for Example 1

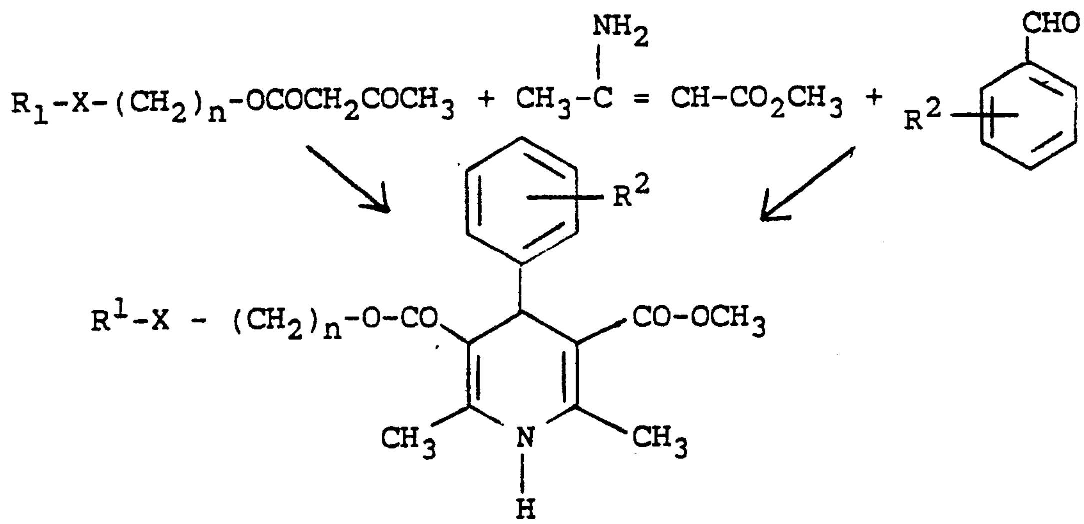

By procedures similar to those used in Example 1, and starting with the appropriately substituted aromatic five-membered heterocyclic alkylacetoacetate and benzaldehyde derivatives, the following compounds were prepared.

**Example 2**

3-[7-(3-methylisoxazol-5-yl)heptyloxycarbonyl]-5-methoxycarbonyl-2,6-dimethyl-4-(2-nitrophenyl)-1,4-dihydropyridine. (2)

$R_1$ = 3-methylisoxazol-5-yl; n = 6; $R_2$ = o-$NO_2$; X = $CH_2$.

Thick yellow oil, (20%), IR (neat) 3335 (NH), 1726 and 1696 ($CO_2R$)$cm^{-1}$; NMR ($CDCl_3$) 1.08 - 1.40 (m, 6H, $(CH_2)_3$), 1.50 - 1.72 (m, 4H, $(CH_2)_2$), 2.28 (S, 3H, Me), 2.30 (S, 3H, Me), 2.36 (S, 3H, Me), 2.96 (t, J = 9Hz, 2H, -$CH_2$-Het), 3.60 (S, 3H, $CO_2Me$), 3.98 - 4.10 (m, 2H, -$CH_2$-O), 5.80 (S, 1H, H-4), 5.85 (S, 1H, H-Het), 6.55 (S, 1H, NH), 7.22 - 7.74 (m, 4H, H-3′, H-4′, H-5′, H-6′). Analysis found: C, 63.25; H, 6.52; N, 8.33. Required: C, 63.39; H, 6.50; N, 8.21 ($C_{27}H_{33}N_3O_7$).

**Example 3**

3-[7-(3-mehylisoxazol-5-yl)heptyloxycarbonyl]-5-methoxycarbonyl-2,6-dimethyl-4-(3-trifluoromethylphenyl)-1,4-dihydropyridine. (3)

$R_1$ = 3-methylisoxazol-5-yl; n = 6; $R_2$ = m-$CF_3$; X = $CH_2$.

Thick oil, (30%); IR (neat) 3330 (NH) 1697 and 1660 ($CO_2R$)$cm^{-1}$. NMR ($CDCl_3$) 1.20 - 1.40 (m, 6H, $(CH_2)_3$), 1.54 - 1.74 (m, 4H, $(CH_2)_2$), 2.30 (S, 3H, Me), 2.36 (S, 3H, Me), 2.38 (S, 3H, Me), 2.72 (t, J=9Hz, 2H, $CH_2$-Het), 3.69 (S, 3H, $CO_2Me$), 3.99 - 4.20 (m, 2H, -$OCH_2$), 5.09 (S, 1H, H-4), 5.86 (S, 1H, H-Het), 6.36 (S, 1H, NH), 7.32 - 7.60 (m, 4H, H-2′, H-4′, H-5′, H-6′). Analysis found: C, 63.05; H, 6.20; N, 5.18. Required: C, 62.91; H, 6.22; N, 5.24 ($C_{28}H_{33}F_3N_2O_5$).

**Example 4**

3-[7-(3-methylisoxazol-5-yl)heptyloxycarbonyl]-5-methoxycarbonyl-2,6-dimethyl-4-(2-trifluoromethylphenyl)-1,4-dihydropyridine. (4)

$R_1$ = 3-methylisoxazol-5-yl; n = 6; $R_2$ = o-$CH_3$; X = $CH_2$.

Thick yellow oil, (22%); IR (neat) 3335 (NH), 1698 and 1686 ($CO_2R$)$cm^{-1}$; NMR ($CDCl_3$) 1.14 - 1.36 (m, 6H, $(CH_2)_3$), 1.50 - 1.70 (m, 4H, $(CH_2)_2$), 2.28 (S, 6H, Me-2, Me-6), 2.32 (S, 3H, Me-Het), 2.69 (t, J = 9Hz, $CH_2$-Het), 3.61 (S, 3H, $CO_2Me$), 3.92 - 4.12 (m, 2H, 0-$CH_2$), 5.57 (S, 1H, H-4), 5.85 (S, 1H, H-Het), 6.55 (S, 1H, NH), 7.20 -7.59 (m, 4H, H-3′, H-4′, H-5′, H-6′). Analysis found: C, 63.05; H, 6.31; N, 5.08. Required: C, 62.91; H, 6.22; N, 5.24 ($C_{28}H_{33}F_3N_2O_5$).

## Example 5

3-[6-(3,5-dimethylpyrazol-1-yl)hexyloxycarbonyl]-5-methoxycarbonyl-2,6-dimethyl-4-(3-nitrophenyl)-1,4-dihydropyridine. (5)

$R_1$ = 3,5-dimethylpyrazol-1-yl; n = 5; $R_2$ = m-$NO_2$; X = $CH_2$.

Thick yellow oil, (45%); IR (neat) 3335 (NH), 1700 and 1688 ($CO_2R$)$cm^{-1}$; NMR ($CDCl_3$) 1.23 - 1.35 (m, 4H, $(CH_2)_2$), 1.60 (q, J=6Hz, 2H, $CH_2$), 1.75 (t, J=6Hz, 2H, $CH_2$), 2.24 (S, 6H, Me-2, Me-6), 2.36 (S, 6H, 2Me-Het), 3.67 (S, 3H, $CO_2Me$), 3.90 - 4.10 (m, 4H, O-$CH_2$ and $CH_2$-Het), 5.11 (S, 1H, H-4), 5.90 (S, 1H, H-Het), 6.75 (S, 1H, NH), 7.34 - 8.16 (m, 4H, H-2′, H-4′, H-5′, H-6′). Analysis found: C, 63.38; H, 6.68; N, 11.13. Required: C, 63.52; H, 6.71; N, 10.97 ($C_{27}H_{34}N_4O_6$).

## Example 6

3-[6-(3,5-dimethylpyrazol-1-yl)hexyloxycarbonyl]-5-methoxycarbonyl-2,6-dimethyl-4-(2-nitrophenyl)-1,4-dihydropyridine. (6)

$R_1$ = 3,5-dimethylpyrazol-1-yl; n = 5; $R_2$ = o-$NO_2$; X = $CH_2$.

Thick yellow oil, (35%); IR (neat) 3340 (NH), 1694 and 1654 ($CO_2R$)$cm^{-1}$; NMR ($CDCl_3$) 1.08 - 1.30 (m, 4H, $(CH_2)_2$), 1.54 (q, JL=6Hz, 2H, $CH_2$), 1.71 (q, J=6Hz, 2H, $CH_2$), 2.23 (S, 6H, Me-3, Me-6), 2.31 (S, 3H, Me-Het), 2.36 (S, 3H, Me-Het), 3.60 (S, 3H, $CO_2Me$), 3.89 - 4.10 (m, 4H, -O$CH_2$ and $CH_2$-Het), 5.79 (S, 2H, H-4 and H-Het), 6.06 (S, 1H, NH), 7.22 - 7.74 (m, 4H, H-3′, H-4′, H-5′, H-6′). Analysis found: C, 63.71; H, 6.79; N, 10.81. Required: C, 63.52; H, 6.71; N, 10.97 ($C_{27}H_{34}N_4O_6$).

## Example 7

3-[6-(3,5-dimethylpyrazol-1-yl)hexyloxycarbonyl]-5-methoxycarbonyl-2,6-dimethyl-4-(3-trifluorophenyl)-1,4-dihydropyridine. (7)

$R_1$ = 3,5-dimethylpyrazol-1-yl; n = 5; $R_2$ = m-$CF_3$; X = $CH_2$.

Thick yellow oil, (40%); IR (neat) 3335 (NH), 1697 and 1687 ($CO_2R$)$cm^{-1}$; NMR ($CDCl_3$) 1.20 - 1.32 (m, 4H, $(CH_2)_2$), 1.56 (q, J=6Hz, 2H, $CH_2$), 1.74 (q, J=6Hz, 2H, $CH_2$), 2.26 (S, 6H, Me-2, Me-6), 2.34 (S, 6H, 2Me-Het), 3.66 (S, 3H, $CO_2Me$), 3.90 - 4.12 (m, 4H, O-$CH_2$ and $CH_2$-Het), 5.06 (S, 1H, H-4), 5.80 (S, 1H, H-Het), 6.62 (S, 1H, NH), 7.28 - 7.56 (m, 4H, H-2′, H-4′, H-5′, H-6′). Analysis found: C, 63.15; H, 6.51; N, 7.75. Required: C, 63.03; H, 6.42; N, 7.88 ($C_{28}H_{34}F_3N_3O_4$).

## Example 8

3-[6-(3,5-dimethylpyrazol-1-yl)hexyloxycarbonyl]-5-methoxycarbonyl-2,6-dimethyl-4-(2-trifluorophenyl)-1,4-dihydropyridine. (8)

$R_1$ = 3,5-dimethylpyrazol-1-yl; n = 5; $R_2$ = o-$CF_3$; X = $CH_2$.

Thick yellow oil, (25%); IR (neat) 3340 (NH), 1700 and 1698 ($CO_2R$)$cm^{-1}$; NMR ($CDCl_3$) 1.10 - 1.30 (m, 4H, $(CH_2)_2$), 1.75 (q, J=6Hz, 2H, -$CH_2$), 2.25 (S, 6H, Me-2, Me-6), 2.30 (S, 6H, 2Me-Het), 3.60 (S, 3H, $CO_2Me$), 3.90 -4.10 (m, 4H, O-$CH_2$, $CH_2$-Het), 5.56 (S, 1H, H-4), 5.80 (S, 1H, H-Het), 6.28 (S, 1H, NH), 7.20 - 7.60 (m, 4H, H-3′, H-4′, H-5′, H-6′). Analysis found: C, 62.91; H, 6.38; N, 8.03. Required: C, 63.03; H, 6.42; N, 7.88 ($C_{28}H_{34}F_3N_3O_4$).

## Example 9

3-[6-(3,5-dimethylpyrazol-1-yl)hexyloxycarbonyl]-5-methoxycarbonyl-2,6-dimethyl-4-(3-chlorophenyl)-1,4-dihydropyridine. (9)

$R_1$ = 3,5-dimethylpyrazol-1-yl; n = 5; $R_2$ = m-Cl; X = $CH_2$.

Thick yellow oil, (50%); IR (neat) 3330 (NH), 1701 and 1655 ($CO_2R$)$cm^{-1}$; NMR ($CDCl_3$) 1.24 - 1.36 (m, 4H, -$(CH_2)_2$), 1.61 (q, J-6Hz, 2H, $CH_2$), 1.76 (q, J = 6Hz, 2H, -$CH_2$), 2.24 (S, 6H, Me-2, Me-6), 2.34 (S, 6H, 2Me-Het), 3.67 (S, 3H, $CO_2Me$), 3.91 - 4.19 (m, 4H, $CH_2$-0 and $CH_2$-Het), 5.01 (S, 1H, H-4), 5.30 (S, 1H, H-Het), 5.81 (S, 1H, NH), 7.08 - 7.30 (m, 4H, H-2′, H′-4′, H-5′, H-6′). Analysis found: C, 65.12; H, 6.69; N, 8.34; Cl, 7.18. Required: C, 64.99; H, 6.67; N, 8.42; Cl, 7.10 ($C_{26}H_{33}ClN_3O_4$).

## Example 10

3-[6-(3,5-dimethylpyrazol-1-yl)hexyloxycarbonyl]-5-methoxycarbonyl-2,6-dimethyl-4-(2-chlorophenyl)-1,4-dihydropyridine. (10)

$R_1$ = 3,5-dimethylpyrazol-1-yl; n = 5; $R_2$ = o-Cl; X = $CH_2$.

Thick yellow oil, (25%); IR (neat) 3335 (NH), 1699 and 1657 ($CO_2R$)$cm^{-1}$; NMR ($CDCl_3$) 1.15 - 1.35 (m, 4H, $(CH_2)_2$), 1.60 (q, J=6Hz, 2H, $CH_2$), 1.75 (q, J-6Hz, 2H, -$CH_2$), 2.26 (S, 6H, Me-2, Me-6), 2.30 (S, 6H, 2Me-Het), 3.65 (S, 3H, $CO_2Me$), 3.90 - 4.10 (m, 4H, $CH_2$-0 and $CH_2$-Het), 5.40 (S, 1H, H-4), 5.80 (S, 1H, H-Het), 6.22 (S, 1H, NH), 7.00 - 7.42 (m, 4H, H-3′, H-4′, H-5′, H-6′). Analysis found: C, 64.85; H, 6.62; N, 8.53; Cl, 7.03. Required: C, 64.99; H, 6.67; N, 8.42; Cl, 7.10 ($C_{27}H_{33}ClN_3O_4$).

## Example 11

3-[7-(4-methylthiazol-2-yl)heptyloxycarbonyl]-5-methoxycarbonyl-2,6-dimethyl-4-(3-nitrophenyl)-1,4-dihydropyridine. (11)

$R_1$ = 4-methylthiazol-2-yl; n = 6; $R_2$ = m-$NO_2$; X = $CH_2$.

Thick yellow oil, (50%); IR (neat) 3330 (NH), 1696 and 1691 ($CO_2R$)$cm^{-1}$; NMR ($CDCl_3$) 1.20 - 1.40 (m, 6H, $(CH_2)_3$), 1.60 (q, J=6Hz, 2H, $CH_2$), 1.75 (q, J=6Hz, 2H, -$CH_2$), 2.35 (S, 6H, Me-2, Me-6), 2.42 (S, 3H, Me-Het), 2.98 (t, J-9Hz, 2H, $CH_2$-Het), 3.66 (S, 3H, $CO_2Me$), 3.96 -4.12 (m, 2H, $CH_2$-O), 5.10 (S, 1H, H-4), 6.54 (S, 1H, H-Het), 6.76 (S, 1H, NH), 7.35 - 8.15 (m, 4H, H-2′, H-4′, H-5′, H-6′). Analysis found: C, 61.35; H, 6.25; N, 8.10; S, 6.00. Required: C, 61.46; H, 6.31; N, 7.96; S, 6.08 ($C_{27}H_{33}N_3O_6S$).

## Example 12

3-[7-(4-methylthiazol-2-yl)heptyloxycarbonyl]-5-methoxycarbonyl-2,6-dimethyl-4-(2-nitrophenyl)-1,4-dihydropyridine. (12)

$R_1$ = 4-methylthiazol-2-yl; n = 6; $R_2$ = o-$NO_2$; X = $CH_2$.

Thick yellow oil, (25%); IR (neat) 3335 (NH), 1698 and 1687 ($CO_2R$)$cm^{-1}$; NMR ($CDCl_3$) 1.20 - 1.40 (m,

6H. $(CH_2)_3$), 1.55 (q, J=6Hz, 2H, $CH_2$), 1.75 (q, J=6Hz, 2H, $CH_2$), 2.30 (S, 3H, Me-6), 2.35 (S, 3H, Me-2), 2.40 (S, 3H, Me-Het), 2.95 (t, J=9Hz, 2H, $CH_2$-Het), 3.60 (S, 3H, $CO_2Me$). 3.95 - 4.10 (m, 2H, $CH_2$-O), 5.80 (S, 1H, H-4), 6.40 (S, 1H, H-Het), 6.74 (S, 1H, NH), 7.20 - 7.75 (m, 4H, H-3′, H-4′, H-5′, H-6′). Analysis found: C, 61.53; H, 6.40; N, 7.78; S, 6.21. Required: C, 61.46; H, 6.31; N, 7.96; S, 6.08 ($C_{27}H_{33}N_3O_6S$).

## Example 13

3-[7-(4-methylthiazol-2-yl)heptyloxycarbonyl]-5-methoxycarbonyl-2,6-dimethyl-4-(3-trifluoromethylphenyl)-1,4-dihydropyridine. (13)

$R_1$ = 4-methylthiazol-2-yl; n = 6; $R_2$ = m-$CF_3$; X = $CH_2$.

Thick yellow oil, (40%); IR (neat) 3335 (NH), 1699 and 1660 ($CO_2R$)$cm^{-1}$; NMR ($CDCl_3$) 1.20 - 1.40 (m, 6H, $(CH_2)_3$), 1.55 (q, J=6Hz, 2H, $CH_2$), 1.76 (q, J=6Hz, 2H, $CH_2$), 2.30 (S, 6H, Me-2, Me-6), 2.42 (S, 3H, Me-Het), 2.98 (t, J=9Hz, 2H, $CH_2$-Het), 3.66 (S, 3H, $CO_2Me$), 3.94 -4.14 (m, 2H, $CH_2$-O), 5.07 (S, 1H, H-4), 6.74 (S, 1H, H-Het), 6.78 (S, 1H, NH), 7.30 - 7.56 (m, 4H, H-2′, H-4′, H-5′, H-6′). Analysis found: C, 60.91; H, 5.96; N, 5.14; S, 5.93. Required: C, 61.08; H, 6.04; N, 5.09; S, 5.82 ($C_{28}H_{33}F_3N_3O_4S$).

## Example 14

3-[7-(4-methylthiazol-2-yl)heptyloxycarbonyl]-5-methoxycarbonyl-2,6-dimethyl-4-(2-trifluoromethylphenyl)-1,4-dihydropyridine. (14)

$R_1$ = 4-methylthiazol-2-yl; n = 6; $R_2$ = o-$CF_3$; X = $CH_2$.

Thick yellow oil, (15%); IR (neat) 3340 (NH), 1700 and 1690 ($CO_2R$)$cm^{-1}$; NMR ($CDCl_3$) 1.12 - 1.42 (m, 6H, $(CH_2)_3$), 1.55 (q, J=6Hz, 2H, $CH_2$), 1.75 (q, J=6Hz, 2H, $CH_2$), 2.22 (S, 6H, Me-2, Me-6), 2.44 (S, 3H, Me-Het), 2.96 (6, J=9Hz, 2H, $CH_2$-Het), 3.62 (S, 3H, $CO_2Me$), 3.92 -4.12 (m, 2H, $CH_2$-O), 5.58 (S, 1H, H-4), 6.06 (S, 1H, H-Het), 6.75 (S, 1H, NH), 7.20 - 7.60 (m, 4H, H-3′, H-4′, H-5′, H-6′). Analysis found: C, 61.15; H, 6.11; N, 4.96; S, 5.70. Required: C, 61.08; H, 6.04; N, 5.09; S, 5.82 ($C_{28}H_{33}F_3N_2O_4S$).

## Example 15

3-[7-(4-methylthiazol-2-yl)heptyloxycarbonyl]-5-methoxycarbonyl-2,6-dimethyl-4-(3-chlorophenyl)-1,4-dihydropyridine. (15)

$R_1$ = 4-methylthiazol-2-yl; n = 6; $R_2$ = m-Cl; X = $CH_2$.

Thick yellow oil, (50%); IR (neat) 3335 (NH), 1698 and 1682 ($CO_2R$)$cm^{-1}$; NMR ($CDCl_3$) 1.20 - 1.44 (m, 6H, $(CH_2)_3$), 1.60 (q, J=6Hz, 2H, $CH_2$), 1.88 (q, J=6Hz, 2H, $CH_2$), 2.34 (S, 6H, Me-2, Me-6), 2.44 (S, 3H, Me-Het), 2.99 (t, J=9Hz, 2H, $CH_2$-Het), 3.68 (S, 3H, $CO_2Me$), 3.96 -4.18 (m, 2H, $CH_2$-O), 5.01 (S, 1H, H-4), 6.76 (S, 2H, H-Het, NH), 7.08 - 7.28 (m, 4H, H-3′, H-4′, H-5′, H-6′). Analysis found: C, 62.58; H, 6.39; N, 5.50; Cl, 6.92; S, 6.13. Required: C, 62.72; H, 6.42; N, 5.42; Cl, 6.86; S, 6.20 ($C_{27}H_{33}ClN_2O_4S$).

## Example 16

3-[7-(4-methylthiazol-2-yl)heptyloxycarbonyl]-5-methoxycarbonyl-2,6-dimethyl-4-(2-chlorophenyl)-1,4-dihydropyridine. (16)

$R_1$ = 4-methylthiazol-2-yl; n = 6; $R_2$ = o-Cl; X = $CH_2$.

Thick yellow oil, (20%); IR (neat) 3335 (NH), 1696 and 1658 ($CO_2R$)$cm^{-1}$; NMR ($CDCl_3$) 1.12 - 1.40 (m, 6H, $(CH_2)_3$), 1.59 (q, J=6Hz, 2H, $CH_2$), 1.76 (q, J=6Hz, 2H, $CH_2$), 2.30 (S, 3H, Me-6), 2.32 (S, 3H, Me-2), 2.44 (S, 3H, Me-Het), 2.98 (t, J=9Hz, 2H, $CH_2$-Het), 3.65 (S, 3H, $CO_2Me$), 3.99 - 4.12 (m, 2H, $CH_2$-O), 5.41

(S, 1H, H-4), 6.12 (S, 1H, H-Het), 6.75 (S, 1H, NH), 7.00 - 7.42 (m, 4H, H-2′, H-4′, H-5′, H-6′). Analysis found: C, 62.88; H, 6.50; N, 5.40; Cl, 8.76; S, 6.05. Required: C, 62.72; H, 6.42; N, 5.42; Cl, 6.86; S, 6.20 ($C_{27}H_{33}ClN_2O_4S$).

## Example 17

3-[7-(4-methylthiazol-2-yl)heptyloxycarbonyl]-5-methoxycarbonyl-2,6-dimethyl-4-(2,3-dichlorophenyl)-1,4-dihydropyridine (17)

$R_1$ = 4-methylthiazol-2-yl; n = 6; $R_2$ = 2,3-dichloro; X = $CH_2$.

Colorless prisms, mp 130 - 131 °C, (25%); IR (KBr) 3340 (NH), 1699 and 1685 ($CO_2R$)$cm^{-1}$; NMR ($CDCl_3$) 1.10 - 1.85 (m, 10H, $(CH_2)_5$), 2.25 (S, 3H, Me-6), 2.30 (S, 3H, Me-2), 2.42 (S, 3H, Me-Het), 2.96 (t, J=9Hz, 2H, $CH_2$-Het), 3.61 (S, 3H, $CO_2Me$), 3.92 - 4.15 (m, 2H, $CH_2$-O), 5.45 (S, 1H, H-4), 6.50 (S, 1H, H-Het), 6.71 (S, 1H, NH), 7.00 - 7.33 (m, 3H, H-4′, H-5′, H-6′). Analysis found: C, 58.92; H, 5.88; N, 4.95; Cl, 13.05; S, 5.74. Required: C, 58.80; H, 5.85; N, 5.08; Cl, 12.86; S, 5.81 ($C_{27}H_{32}Cl_2N_2O_4S$).

## Example 18

3-[6-(4-methylthiazol-2-yl)thiohexyloxycarbonyl]-5-methoxycarbonyl-2,6-dimethyl-4-(3-nitrophenyl)-1,4-dihydropyridine. (18)

$R_1$ = 4-methylthiazol-2-yl; n = 6; $R_2$ = m-$NO_2$; X = S.

Yellow oil (80%); IR (neat) 3320 (NH), 1700 and 1685 ($CO_2R$)$cm^{-1}$ ; NMR ($CDCl_3$) 1.20 - 1.80 (m, 8H, $(CH_2)_4$), 2.36 (S, 6H, Me-2, Me-6), 2.42 (S, 3H, Me-Het), 3.16 (t, J=9Hz, 2H, $CH_2$-S), 3.66 (S, 3H, $CO_2Me$), 4.00 -4.18 (m, 2H, $CH_2$-O), 5.14 (S, 1H, H-4), 6.68 (S, 1H, H-Het), 6.80 (S, 1H, NH), 7.36 - 8.16 (m, 3H, H-2′, H-4′, H-5′, H-6′). Analysis found: C, 57.38; H, 5.80; N, 7.65; S, 11.68. Required: C, 57.23; H, 5.73; N, 7.70; S, 11.75 ($C_{26}H_{31}N_3O_6S_2$).

## Example 19

3-[6-(4-methylthiazol-2-yl)sulfonylhexyloxycarbonyl]-5-methoxycarbonyl-2,6-dimethyl-4-(3-nitrophenyl)-1,4-dihydropyridine. (19)

$R_1$ = 4-methylthiazol-2-yl; n = 6; $R_2$ = m-$NO_2$; X = $SO_2$.

Colorless prisms, mp 102 - 105 °C (75%); IR (KBr) 3375 (NH), 1698 and 1669 ($CO_2$)$cm^{-1}$; NMR ($CDCl_3$) 1.18 - 1.84 (m, 8H, $(CH_2)_4$), 2.32 (S, 6H, Me-2, Me-6), 2.52 (S, 3H, Me-Het), 3.35 (t, J=9Hz, 2H, $CH_2$-$SO_2$), 3.65 (S, 3H, $CO_2Me$), 3.90 - 4.10 (m, 2H, $CH_2$-O), 5.10 (S, 1H, H-4), 6.48 (S, 1H, H-Het), 7.34 - 8.10 (m, 5H, NH, H-2′, H-4′, H-5′, H-6′ . Analysis found: C, 54.20; H, 5.50; N, 7.15; S, 11.00. Required: C, 54.06; H, 5.41; N, 7.27; S, 11.10 ($C_{26}H_{31}N_3O_8S_2$).

## Example 20

3-[6-(4-methylthiazol-2-yl)sulfoxyhexyloxycarbonyl]-5-methoxycarbonyl-2,6-dimethyl-4-(3-nitrophenyl)-1,4-dihydropyridine. (20)

$R_1$ = 4-methylthiazol-2-yl; n = 6; $R_2$ = 3-$NO_2$; X = SO.

(See schematic representation of reaction given herebelow)

A solution of m-chloroperbenzoic acid (0.156g, 0.001 mol) in dichloromethane (5mL) was added to a solution of 3-[6-(4-methylthiazol-2-yl) thiohexyloxycarbonyl]-5-methoxycarbonyl-2,6-dimethyl-4-(3-nitrophenyl)-1,4-dihydropyridine (18) (0.545g, 0.001 mol) in dichloromethane (10mL) at room temperature

with agitation. Stirring under the same conditions was maintained for 2h. The excess peracid was decomposed by 10% solution of sodium sulfite. The organic layer was separated and washed with 5% sodium bicarbonate solution in order to extract m-chlorobenzoic acid, followed by washing with water and drying over anhydrous sodium sulfate. The solvent was removed under reduced pressure, and the residual oil was purified by elution from a silica gel column using chloroform-hexane (8:2, v/v) as eluant to give 3-[6-(4-methylthiazol-2-yl) sulfoxyhexyloxycarbonyl]-5-methoxycarbonyl-2,6-dimethyl-4-(3-nitrophenyl)-1,4-dihydropyridine (65%) as colorless prisms, mp 81 - 83° C. IR (KBr) 3365 (NH) 1695 and 1652 ($CO_2R$) 1033 (SO)$cm^{-1}$, NMR ($CDCl_3$) 1.22 - 1.74 (m, 8H, $(CH_2)_4$), 2.36 (S, 6H, Me-2, Me-6) 2.52 (S, 3H, Me-HEt),3.02 - 3.22 (m, 2H, $CH_2$-SO), 3.68 (S, 3H, $CO_2Me$), 3.96 - 4.14 (m, 2H, $CH_2$-O), 5.12 (S, 1H, H-4), 6.56 (S, 1H, H-HEt), 7.26 (S, 1H, NH), 7.38 - 8.14 (m, 4H, H-2′, H-4′, H-5′, H-6′). Analysis found: C, 55.75; H, 5.64; N, 7.35; S, 11.30. Required: C, 55.66; H, 5.56; N, 7.48; S, 11.42 ($C_{26}H_{31}N_3O_7S_2$).

Schematic representation of reaction

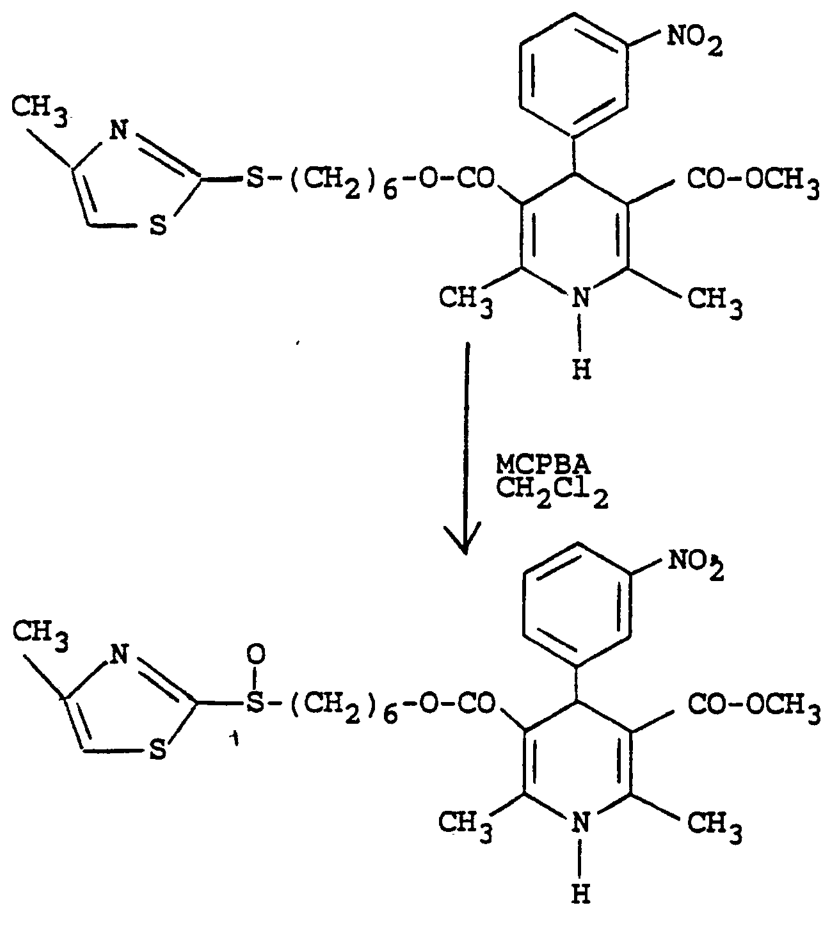


**Example 21**

5-(7-Acetoacetoxyhept-1-yl)-3-methylisoxazole. (21)

(See schematic representation of the reaction given herebelow).

To a mixture of 7-(3-methylisoxazol-5-yl)heptan-1-ol (1.97g, 0.01 mol) and anhydrous sodium acetate (0.080g) in benzene (20mL) was added a solution of diketene (1.84g, 0.022 mol) in benzene (6mL) dropwise. The mixture was refluxed for 12h. After the removal of benzene under reduced pressure, the crude product was purified by elution from a silica gel column using dichloromethane-hexane (9:1 v/v) as eluant to give 5-(7- acetoacetoxyhept-1-yl)-3-methylisoxazole (21) (70%) as a yellow oil. IR (neat) 1740

($CO_2$), 1718 (CO)$cm^{-1}$; NMR ($CDCl_3$) 1.12 - 1.95 (m, 10H, $(CH_2)_5$), 2.35 (S, 6H, Me-Het, CO-Me), 2.70 (t, J=9Hz, 2H, $CH_2$-Het), 3.49 (S, 2H, CO-$CH_2$-COMe), 4.14 (t, J=6Hz, 2H, O-$CH_2$), 5.80 (S, 1H, H-Het). Analysis found: C, 64.11; H, 8.31; N, 4.72. Required: C, 64.02; H, 8.24; N, 5.00 ($C_{15}H_{23}NO_4$).

Schematic of Example 21

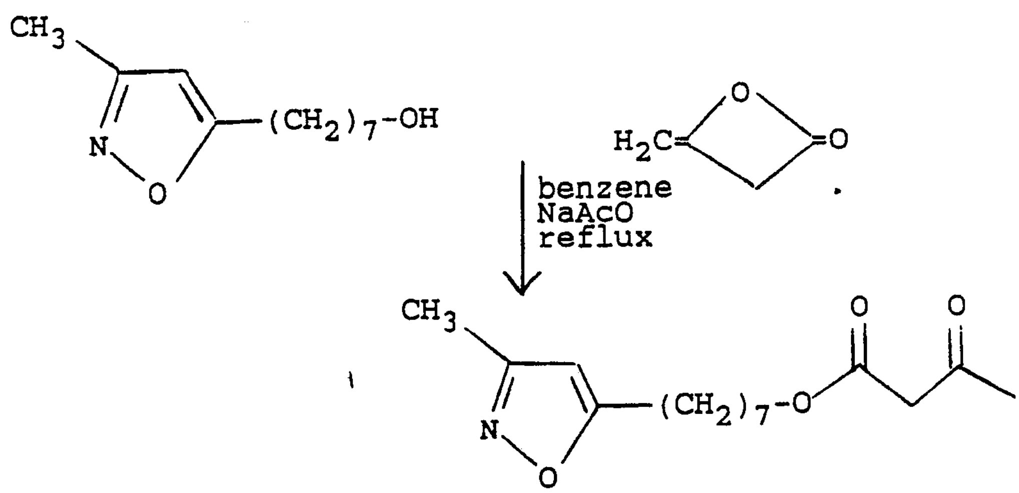


Following the same procedure as Example 21, and starting with the appropriately substituted ω-heterocyclic alkanol, the following compounds were prepared.

**Example 22**

1-(6-Acetoacetoxyhex-1-yl)-3,5-dimethylpyrazole. (22)

Yellow oil (75%). IR (neat) 1741($CO_2$) 1718(CO)$cm^{-1}$; NMR ($CDCl_3$) 1.20 - 2.10 (m, 8H, $(CH_2)_4$), 2.30 (S, 9H, Me-3, Me-5, -COMe), 3.50 (S, 2H, CO-$CH_2$-CO), 3.88 - 4.32 (m, 4H, $CH_2$N, -$CH_2$O), 5.81 (S, 1H, H-Het). Analysis found: C, 64.29; H, 8.71; N, 9.85. Required: C, 64.23; H, 8.62; N, 10.04 ($C_{15}H_{24}N_2O_3$).

**Example 23**

2-(7-Acetoacetoxyhept-1-yl)-4-methylthiazole. (23)

Yellow oil (80%). IR (neat) 1740($CO_2$), 1716(CO)$cm^{-1}$; NMR ($CDCl_3$) 1.30 - 1.50 (m, 6H, $(CH_2)_3$), 1.65 (q, J=6Hz, 2H, $CH_2$), 1.78 (q, J=6Hz, 2H, -$CH_2$), 2.28 (S, 3H, Me-Het), 2.40 (S, 3H, $CH_3$-CO), 2.96 (t, J=9Hz, 2H, -$CH_2$-Het), 3.46 (S, 2H, CO-$CH_2$-CO), 4.15 9t, J=9Hz, 2H, -$CH_2$O), 6.72 (S, 1H, H-Het). Analysis found: C, 60.68; H, 7.83; N, 4.21; S, 10.55. Required: C, 60.56; H, 7.79; N, 4.73; S, 10.78 ($C_{15}H_{23}NO_3S$).

**Example 24**

2-(6-Acetoacetoxyhexylthio)-4-methylthiazole. (24)

Yellow oil (99%). IR (neat) 1742($CO_2$), 1715(CO)$cm^{-1}$; NMR ($CDCl_3$) 1.25 - 2.10 (m, 8H, $(CH_2)_4$), 2.23 (S, 3H, Me-Het), 2.36 (S, 3H, CO-$CH_3$), 3.16 (t, J=9Hz, 2H, -$CH_2$-S), 3.46 (S, 2H, CO-$CH_2$-CO), 4.14 (t, J=9Hz, 2H, -$CH_2$-O), 6.77 (S, 1H, H-Het). Analysis found: C, 53.35; H, 6.79; N, 4.23; S, 20.23. Required: C,

53.30; H, 6.71; N, 4.46; S, 2032 ($C_{14}H_{21}NO_3S_2$).

**Example 25**

2-(6-Acetoacetoxyhexylsulfonyl)-4-methylthiazole. (25)

(See the schematic representation of reaction given herebelow)

2-(6-Acetoacetoxyhexylthio)-4-methylthiazole (3.15g, 0.01 mol) was dissolved in a mixture of glacial acetic acid (125mL) and water (25mL) at room temperature. To the mixture was added fractionwise (3.32g, 0.021 mol) of $KMnO_4$ while stirring, keeping the temperature at approximately 25° C for 2h. The mixture was stirred at room temperature for an additional 1-1/2h, then decolorized with $H_2O_2$ (30%). Ice water (200mL) was added to the mixture and filtered. The filtrate was extracted with diethyl ether. After the removal of the solvent, under reduced pressure, the crude product was purified by elution from a silica gel column using chloroform-hexane (8:2 v/v) as eluant to give 2-(6-Acetoacetoxyhexylsulfonyl)-4-methylthiazole (25) (25%) as a yellow oil. IR (neat) 1741 ($CO_2$), 1719(-CO)$cm^{-1}$; NMR ($CDCl_3$) 1.18 - 2.00 (m, 8H, $(CH_2)_4$), 2.26 (S, 3H, Me-Het), 2.55 (S, 3H, $CO_2Me$), 3.20 - 3.56 (m, 4H, $-CH_2-SO_2$ and $CO-CH_2-CO$), 3.96 - 4.23 (t, J=9Hz, 3H, $-CH_2-$:O), 7.34 (S, 1H, H-Het). Analysis found: C, 48.46; H, 6.17; N, 3.87; S, 18.37. Required: C, 48.39; H, 6.09; N, 4.05; S, 18.45 ($C_{14}H_{21}NO_5S_2$).

Schematic for Example 25

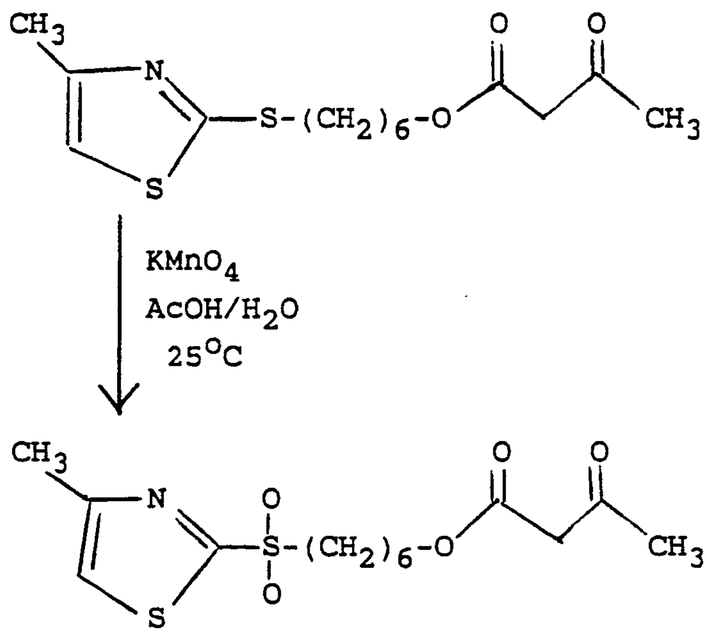


**Example 26**

7-(3-Methylisoxazol-5-yl)heptan-1-ol. (26)

(See the schematic representation of reaction given herebelow)

To a solution of diisopropylamine (9.8mL, 0.07 mol) in tetrahydrofuran (17.5mL) at -5° C and under nitrogen, was added n-Butyllithium (1.6M) in hexane (23.1mL, 0.78 mol) dropwise keeping the temperature constant. After the addition was complete, the solution was cooled to -60° C and 3,5-dimethylisoxazole (2.91g, 0.03 mol) in THF (7.5mL) was added dropwise. The mixture was stirred for an additional 1h at

-60° C, then added via a nitrogen purge to 6-bromo-1-hexanol (3.62g, 0.02 mol) in THF (10mL), and chilled to -60° C while stirring. The mixture was allowed to gradually warm to room temperature and then stirred overnight.

After quenching with saturated $NH_4Cl$ solution (7mL), the mixture was extracted with chloroform (175mL) and the extract was washed with water and dried. Following the removal of solvent and unreacted 6-bromo-1-hexanol by distillation, the residue was purified by elution from a silica gel column using $CH_2Cl_2$:hexane (9:1) as eluant to give 7-(3-methylisoxazol-5-yl)heptan1-ol (26) (50%) as a yellow oil. IR (neat) 3610 (OH)$cm^{-1}$; NMR ($CDCl_3$) 1.12 - 1.96 (m, 10H, $(CH_2)_5$), 2.26 (S, 3H, Me-Het), 2.66 (t, J=9Hz, 2H, $CH_2$-Het), 3.58 (t, J=9Hz, 2H, $CH_2$-O), 5.84 (S, 1H, H-Het). Analysis found: C, 67.10; H, 9.79; N, 7.02. Required: C, 66.95; H, 9.71; N, 7.13 ($C_{11}H_{19}NO_2$).

## Schematic for Example 26

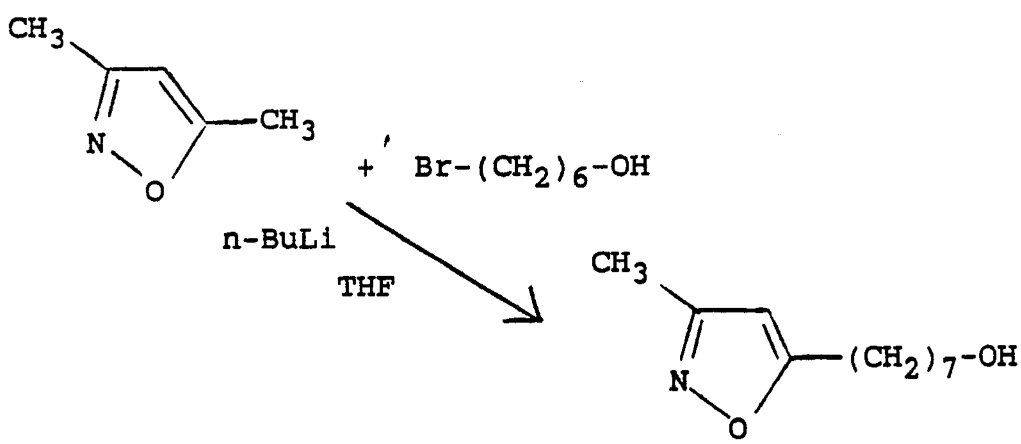


Following the same procedure as example 26, and starting with the appropriately substituted five-membered aromatic heterocycle, the following compounds were prepared.

### Example 27

6-(3,5-dimethylpyrazol-1-yl)hexan-1-ol. (27)

Yellow oil (70%). IR (neat) 3590 (OH)$cm^{-1}$; NMR ($CDCl_3$) 1.10 - 1.95 (m, 8H, $(CH_2)_4$), 2.20 (S, 6H, Me-3, Me-5), 2.89 (S, 1H, OH), 3.49 - 4.10 (m, 4H, $CH_2$-N, $CH_2$-0), 5.72 (S, 1H, H-Het). Analysis found: C, 67.33; H, 10.32; N, 14.25. Required: C, 67.26; H, 10.26; N, 14.33 ($C_{11}H_{20}N_2O$).

### Example 28

7-(4-methylthiazol-2-yl)heptan-1-ol. (28)

Yellow oil (50%). IR (neat) 3605 (OH)$cm^{-1}$; NMR ($CDCl_3$) 1.30 - 1.75 (m, 10H, $(CH_2)_5$), 2.40 (S, 3H, Me-Het), 2.95 (t, J=9Hz, $CH_2$-Het), 3.60 (t, J=9Hz, 2H, $CH_2$-O), 4.45 (S, 1H, H), 6.70 (S, 1H, H-Het). Analysis found: C, 62.03; H, 9.07; N, 6.47; S, 14.96. Required: C, 61.91; H, 8.97; N, 6.60; S, 15.02 ($C_{11}H_{19}NOS$).

### Example 29

6-(4-methylthiazol-2-yl)thiohexan-1-ol. (29)

(See the schematic representation of reaction given herebelow)

The mixture of 2-mercapto-4-methylthiazole (2.62g, 0.02 mol), 6-bromo-1-hexanol (3.62g, 0.02 mol), potassium carbonate (2.76g, 0.02 mol) and acetone (50mL) was refluxed for 14h. After cooling down, the mixture was filtered and the filtrate was evaporated. The residual oil was purified by elution from a silica gel column using ethylacetate-methanol (99:1, v/v) as eluant to give 6-(4-methylthiazol-2-yl)thiohexan-1-ol (29) (99%) as a yellowish oil. IR (neat) 3608 (OH)$cm^{-1}$; NMR ($CDCl_3$) 1.25 - 2.10 (m, 8H, $(CH_2)_4$), 2.38 (S, 3H, Me-Het), 3.18 (t, J=9Hz, 2H, $CH_2$-S), 3.65 (t, J=9Hz, 2H, $CH_2$-O), 6.75 (S, 1H, H-Het). Analysis found: C, 51.98; H, 7.49; N, 5.92; S, 27.65. Required: C, 51.90; H, 7.40; N, 6.08; S, 27.71 ($C_{10}H_{17}NOS_2$).

Schematic for Example 29

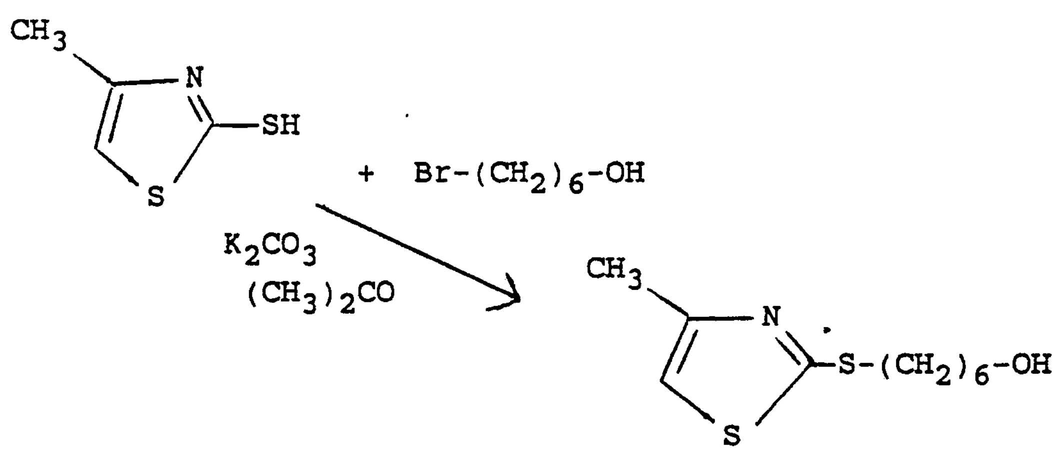


Calcium Channel Antagonist Testing

Calcium channel antagonist activity was determined as the concentration required to produce 50% inhibition of the muscarinic receptor-mediated $Ca^{+2}$-dependent contraction of guinea pig ileal longitudinal smooth muscle assay (C. Triggle, V. Swamy and D. Triggle, Can. J. Physiol. Pharmacol., 1979, 57, 804). Male albino guinea pigs (body weight 300 - 450g) were sacrificed by decapitation. The intestine was removed above the ileocaecal junction. Longitudinal smooth muscle segments of 2cm length were mounted under a resting tension of 300 -400 mg. The segments were maintained at 37° C in a 10mL jacketed organ bath containing oxygenated (100% $O_2$) physiological saline solution of the following composition (mM): NaCl: 137; $CaCl_2$: 2.6; KCl: 5.9; $MgCl_2$: 1.2; glucose: 11.9; buffered by Hepes-NaOH to pH 7.4. The muscles were equilibrated for 1h with a solution change every 15 min. Two successive control contractions were elicited at 15 min. intervals with $5x10^{-7}$M carbachol. The isometric contractions were recorded with a force displacement transducer (FT 03C) on a GRASS * physiograph. The means of the three contractile responses was taken as the 100% value for the tonic (slow) component of the response. The muscle was washed with Hepes saline solution and was allowed to re-equilibrate. The calcium antagonist (test compound) was added ten min before the dose-response for carbachol was determined. The drug-induced inhibition of contraction was expressed as percent of control. The $ID_{50}$ values were graphically determined from the concentration-response curves, in triplicate. The test results are shown in Table 1.

Table 1

| Calcium channel antagonist antihypertensive activity for the 3-(heteroarylalkyloxycarbonyl)-5-methoxycarbonyl-2,6-dimethyl-4-(2 or 3-substitutedphenyl)-1,4-dihydropyridine derivative tested. | | |
|---|---|---|
| | calcium channel antagonist act., inhib. act. on contractile | |
| **Compound No.** | **Response to CD $ID_{50}$(M)** | |
| 1 | 3.84 | $0.81 \times 10^{-9}$ |
| 3 | 5.13 | $1.09 \times 10^{-9}$ |
| 5 | 6.24 | $0.46 \times 10^{-9}$ |
| 11 | 3.25 | $0.33 \times 10^{-9}$ |
| 15 | 6.25 | $0.20 \times 10^{-9}$ |
| 18 | 5.48 | $0.74 \times 10^{-9}$ |
| 20 | 1.54 | $0.09 \times 10^{-8}$ |
| 19 | 5.81 | $0.13 \times 10^{-9}$ |
| 17 | 4.81 | $0.25 \times 10^{-8}$ |
| Nifedipine | 1.40 | $0.20 \times 10^{-8}$ |

The above-mentioned compounds did not show any negative ionotropic effect on heart muscle. Compound 11 has shown about 12% positive ionotropic effect on heart muscle with the same concentration as a smooth muscle relaxant ($3.25 \times 10^{-9}$M). In the same condition, Compound 19 showed 25% positive ionotropic effect on heart muscle. Heart rate did not change in most of the cases. In SHR Rat, Compound 11 at a concentration of 0.2mg/Kg showed a 19% drop in blood pressure for a period of 9h without changing the heart rate.

The in-vitro angiotnesine inhibitory activity of certain compounds is shown in Table II given herebelow.

Table 2

| Example No. | Concentration µg/ml | Percent Inhibition |
|---|---|---|
| 1 | 0.01 | 93 |
| 5 | 0.01 | 95 |
| 13 | 0.1 | 80 |
| 15 | 0.1 | 64 |
| Captopril | 0.05 | 69 |

Captopril * which is the reference compound marketed by Squibb under the brand name Captoten * , is known as an angiotensin converting enzyme inhibitor.

The compound of Example 1 at a concentration of 0.0025 µg still shows 60 percent inhibition. The compound of Example 5 at a concentration of 0.001 µg shows 58 percent inhibition.

## Claims

* Trade Mark

1. A 1,4-dihydropyridine derivative of the formula

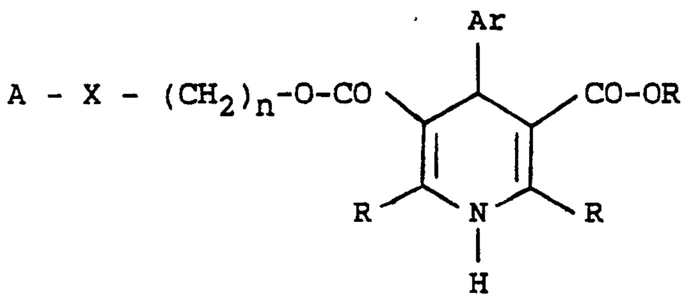

(I)

is provided in which:
A is an optionally substituted non-fused azole moiety;
R is a lower alkyl group;
X is $-CH_2-$ , -S- , -SO- or $-SO_2-$ ;
n is 5, 6, 7 or 8; and
Ar is a phenyl group substituted once or twice by $NO_2$, $CF_3$ or Cl groups.

2. A 1,4-dihydropyridine derivative of the formula I as claimed in Claim 1, wherein each group R is a methyl group and A is 3-methyl-5-isoxazolyl, 3,5-dimethyl-1-pyrazolyl, or 4-methyl-2-thiazolyl.

3. A compound as claimed in Claim 1 or 2 wherein A is 3-methyl-5-isoxazolyl.

4. A compound as claimed in Claim 1 or 2 wherein A is 3,5-dimethyl-1-pyrazolyl.

5. A compound as claimed in Claim 1 or 2 wherein A is 4-methyl-2-thiazolyl.

6. A compound as claimed in Claim 1 or 2 wherein A is 3-methyl-5-isoxazolyl, 3,5-dimethyl-1-pyrazolyl, or 4-methyl-2-thiazolyl, X is $CH_2$ or $-SO_2-$, n is 6 and Ar is a phenyl group substituted once or twice by $NO_2$, or once by $CF_3$.

7. A compound as claimed in Claim 1 or 2, selected from:
3-[7-(3-methylisoxazol-5-yl)heptyl-oxycarbonyl]-5-methoxycarbonyl-2,6-dimethyl-4-(3-nitrophenyl)-1,4-dihydropyridine;
3-[7-(3-methylisoxazol-5-yl)heptyl-oxycarbonyl]-5-methoxycarbonyl-2,6-dimethyl-4-(2-nitrophenyl)-1,4-dihydropyridine;
3-[7-(3-methylisoxazol-5-yl)heptyl-oxycarbonyl]-5-methoxycarbonyl-2,6-dimethyl-4-(3-trifluoromethylphenyl)-1,4-dihydropyridine;
3-[7-(3-methylisoxazol-5-yl)heptyl-oxycarbonyl]-5-methoxycarbonyl-2,6-dimethyl-4-(2-trifluoromethylphenyl)-1,4-dihydropyridine;
3-[6-(3,5-dimethylpyrazol-1-yl)hexyloxycarbonyl]-5-methoxycarbonyl-2,6-dimethyl-4(3-nitrophenyl)-1,4-dihydropyridine;
3-[6-(3,5-dimethylpyrazol-1-yl)hexyloxycarbonyl]-5-methoxycarbonyl-2,6-dimethyl-4(2-nitrophenyl)-1,4-dihydropyridine;
3-[6-(3,5-dimethylpyrazol-1-yl)hexyloxycarbonyl]-5-methoxycarbonyl-2,6-dimethyl-4(3-trifluoromethylphenyl)-1,4-dihydropyridine;
3-[6-(3,5-dimethylpyrazol-1-yl)hexyloxycarbonyl]-5-methoxycarbonyl-2,6-dimethyl-4(2-trifluoromethlphenyl)-1,4-dihydropyridine;
3-[6-(3,5-dimethylpyrazol-1-yl)hexyloxycarbonyl]-5-methoxycarbonyl-2,6-dimethyl-4(3-chlorophenyl)-1,4-dihydropyridine;
3-[6-(3,5-dimethylpyrazol-1-yl)hexyloxycarbonyl]-5-methoxycarbonyl-2,6-dimethyl-4(2-chlorophenyl)-1,4-dihydropyridine;
3-[7-(4-methylthiazol-2-yl)heptyloxycarbonyl]-5-methoxycarbonyl-2,6-dimethyl-4-(3-nitrophenyl)-1,4-dihydropyridine;
3-[7-(4-methylthiazol-2-yl)heptyloxycarbonyl]-5-methoxycarbonyl-2,6-dimethyl-4-(2-nitrophenyl)-1,4-dihydropyridine;
3-[7-(4-methylthiazol-2-yl)heptyloxycarbonyl]-5-methoxycarbonyl-2,6-dimethyl-4-(3-trifluoromethylphenyl)-1,4-dihydropyridine;
3-[7-(4-methylthiazol-2-yl)heptyloxycarbonyl]-5-methoxycarbonyl-2,6-dimethyl-4-(2-trifluoromethylphenyl)-1,4-dihydropyridine;
3-[7-(4-methylthiazol-2-yl)heptyloxycarbonyl]-5-methoxycarbonyl-2,6-dimethyl-4-(3-chlorophenyl)-1,4-dihydropyridine;
3-[7-(4-methylthiazol-2-yl)heptyloxycarbonyl]-5-methoxycarbonyl-2,6-dimethyl-4-(2-chlorophenyl)-1,4-dihydropyridine;
3-[7-(4-methylthiazol-2-yl)heptyloxycarbonyl]-5-methoxycarbonyl-2,6-dimethyl-4-(2,3-dichlorophenyl)-1,4-dihydropyridine;
3-[6-(4-methylthiazol-2-yl)thiohexyloxycarbonyl]-5-methoxycarbonyl-2,6-dimethyl-4-(3-nitrophenyl)-1,4-dihydropyridine;
3-[6-(4-methylthiazol-2-yl)sulfoxyhexyloxycarbonyl]-5-methoxycarbonyl-2,6-dimethyl-4-(3-nitrophenyl)-1,4-dihydropyridine;
3-[6-(4-methylthiazol-2-yl)sulfonylhexyloxycarbonyl]-5-methoxycarbonyl-2,6-dimethyl-4-(3-nitrophenyl)-1,4-dihydropyridine.

8. A compound as claimed in Claim 1 or 2, wherein A is 3-methyl-5-isoxazolyl, 3,5-dimethyl-1-pyrazolyl, or 4-methyl-2-thiazolyl, X is -S- or -SO-, n is 6 and Ar is a phenyl group substituted once or twice by $NO_2$, or once by $CF_3$.

9. A process for the preparation of a compound of formula I, as claimed in any preceding claim, which process comprises treating a compound of formula III

$A\text{-}X\text{-}(CH_2)_n\text{-}O\text{-}CO\text{-}CH_2\text{-}O\text{-}R$ (III)

wherein A, X and n are as defined by any of the preceding claims, with an alkyl-$\beta$-aminoalkylcrotonate and a compound of formula IV

Ar-CHO (IV)

and optionally oxidising a resultant compound of formula I in which -X- is -S- or -SO- to generate a higher oxide.

10. A process for the preparation of a compound of formula I, as claimed in Claim 9, in which compound each group R is methyl, which process comprises treating a compound of formula III

$A\text{-}X\text{-}R^1\text{-}(CH_2)_n\text{-}O\text{-}CO\text{-}CH_2\text{-}O\text{-}CH_3$ (III)

wherein A, X and n are as defined by any of Claims 1 to 8, with methyl-$\beta$-aminocrotonate and a compound of formula IV

Ar-CHO (IV)

and optionally oxidising a resultant compound of formula I in which -X- is -S- or -SO- to generate a higher oxide.

11. A compound of formula III, as defined by Claim 9 or 10.

12. A compound of formula III, as claimed in Claim 11, selected from:
5-(7-acetoacetoxyhept-1-yl)3-methylisoxazol;
1-(6-acetoacetoxyhex-1-yl)-3,5-dimethylpyrazol;
2-(7-acetoacetoxyhept-1-yl)-4-methylthiazol;
2-(6-acetoacetoxyhex-1-ylthio)-4-methylthiazol;
2-(6-acetoacetoxyhex-1-ylsulfonyl)-4-methylthiazol;

13. A process for the preparation of a compound of formula III as claimed in Clam 11 or 12, which process comprises reacting diketene or a (lower alkyl) diketene with a compound of the formula V

$A\text{-}X^1\text{-}(CH_2)_n\text{-}OH$ (V)

where A and n are as defined by any previous claim, and $X^1$ represents $-CH_2-$ or -S-, and, if a compound in which X represents -SO- or $-SO_2-$ is required, oxidising an appropriate compound in which $X^1$ is -S-.

14. A compound of formula V, as defined by claim 13.

15. A compound of formula V, as claimed in Claim 14, selected from:
7-(3-methylisoxazol-5-yl)heptan-1-ol;
6-(3,5-dimethylpyrazol-1-yl)hexan-1-ol;
7-(4-methylthiazol-2-yl)heptan-1-ol;
6-(4-methylthiazol-2-yl)thiohexan-1-ol;

16. A composition comprising a compound as claimed in any of Claims 1 to 8, and a pharmaceutically acceptable carrier.

17. A compound as claimed in any of Claims 1 to 8, or a composition as claimed in Claim 16, for use as an active therapeutic agent.

18. A compound as claimed in any of Claims 1 to 8, or a composition as claimed in Claim 16, for use in one or more of the following methods of treatment of a patient:
a method of regulating cardiovascular activity;
a method of treating hypertension;
a method for inhibiting angiotensin release;
a method of treating inflammatory activity;

a method of treating an ulcer or of inhibiting ulcer formation.

European Patent Office

# EUROPEAN SEARCH REPORT

Application number

EP 90300478.6

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| A | WO - A1 - 87/05 512 (NELSON RESEARCH) * Formula I; claim 4 * -- | 1 | C 07 D 401/12<br>A 61 K 31/435 |
| A | DE - A1 - 3 607 821 (BAYER) * Examples 15-17 * -- | 1 | |
| D,A | DE - A1 - 3 230 400 (TOKYO TANABE CO) * Claims 1-24 * -- | 1,11, 14 | |
| A | CHEMICAL ABSTRACTS, vol. 104, no. 25, June 23, 1986, Columbus, Ohio, USA ISHIHAMA, HIROSHI et al.: "Imidazolylalkyl 1,4-dihydro-pyridinecarboxylates." page 616, column 1, abstract-no. 224 898c & Jpn. Kokai Tokkyo Koho JP 60,226,876 (85,226,876) -- | 1 | TECHNICAL FIELDS SEARCHED (Int Cl 5) |
| A | CHEMICAL ABSTRACTS, vol. 102, no. 13, April 1, 1985, Columbus, Ohio, USA TOKYO TANABE CO., LTD.: "1,4-Dihydropyridine deriva-tives." page 714, column 1, abstract-no. 113 481a & Jpn. Kokai Tokkyo Koho JP 59,148 779 (84,148,779) ---- | 1 | C 07 D 401/00 |

The present search report has been drawn up for all claims

| Place of search | Date of completion of the search | Examiner |
|---|---|---|
| VIENNA | 07-03-1990 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82